# EUROPÄISCHE PATENTSCHRIFT

(11) **EP 2 144 641 B1**
(45) Veröffentlichungstag und Bekanntmachung des Hinweises auf die Patenterteilung: **11.07.2012**
(21) Anmeldenummer: 08749462.1
(22) Anmeldetag: 15.05.2008
(51) Int. Cl.: A61L 31/10, A61L 31/14

(54) **GEFÄSSSTENT**
VESSEL STENT
STENT

(30) Priorität: 16.05.2007 DE 102007024256
(43) Veröffentlichungstag der Anmeldung: 20.01.2010
(73) Patentinhaber: GELITA AG, 69412 Eberbach (DE)
(72) Erfinder: FLECHSENHAR, Klaus, 69412 Eberbach (DE); AHLERS, Michael, 69412 Eberbach (DE)
(74) Vertreter: Hoeger, Stellrecht & Partner Patentanwälte
(86) Internationale Anmeldenummer: PCT/EP2008/003894
(87) Internationale Veröffentlichungsnummer: WO 2008/138611

(56) Entgegenhaltungen:
- EP-A- 0 237 037
- US-A- 5 632 776
- US-A1- 2007 077 274
- SHIROTA T ET AL: "Fabrication of endothelial progenitor cell (EPC)-seeded intravascular stent devices and in vitro endothelialization on hybrid vascular tissue" BIOMATERIALS, ELSEVIER SCIENCE PUBLISHERS BV., BARKING, GB, Bd. 24, Nr. 13, 1. Juni 2003 (2003-06-01), Seiten 2295-2302, XP004420020 ISSN: 0142-9612

## Beschreibung

Die vorliegende Erfindung betrifft einen Gefäßstent, insbesondere einen koronaren Stent.

Stents werden in der Medizin als Implantate eingesetzt, um Hohlstrukturen von Organen im Körper offenzuhalten bzw. nach einem Verschluss (Stenose) wieder zu öffnen. In der Regel wird dabei ein röhrchenförmiger Stent in einer komprimierten Form in das betreffende Organ eingebracht und anschließend expandiert, um die Wände des Organs abzustützen.

Besondere Bedeutung haben Stents bei der Öffnung von Blutgefäßen, und hier insbesondere im Bereich der Herzkranzgefäße (koronare Stents). Die Verengung oder der Verschluss von Herzkranzgefäßen durch die Ablagerung von Thromben (Blutgerinnseln), Blutfetten oder Kalk an den Gefäßwänden ist eine häufige Ursache für die Unterversorgung von Bereichen des Herzmuskels mit Blut und damit für einen Herzinfarkt. Die zur Vermeidung oder Beseitigung derartiger Stenosen eingesetzten Stents bestehen häufig aus einem röhrchenförmigen Gittergerüst, welches in das betroffene Gefäß eingeführt und mittels eines Ballon-Katheters radial expandiert wird, um das Gefäß wieder auf eine ausreichende Größe zu weiten.

Bei derartigen Behandlungen mit Gefäßstents besteht jedoch regelmäßig das Problem der so genannten Restenose. Hierunter versteht man eine erneute Verengung des Gefäßes durch Ablagerungen auf dem Stent und/oder die Überwucherung des Stents mit Gewebe, die durch eine Aktivierung von Blutplättchen und Gerinnungsfaktoren am "Fremdmaterial" des Stents (in der Regel Metall oder Kunststoff) induziert wird. Eine Restenose macht häufig eine erneute Behandlung des betroffenen Gefäßes erforderlich.

Zur Vermeidung oder Reduzierung dieses Problems werden seit einiger Zeit medikamentenbeschichtete Stents (drug-eluting stents, DES) eingesetzt. Diese Stents weisen eine Beschichtung auf, aus der bestimmte Wirkstoffe zur Hemmung der Gewebebildung freigesetzt werden (z.B. Antiproliferativa, Cytostatika oder Immunsupressiva). Durch die Verwendung derartiger Stents soll das Risiko einer Restenose des betreffenden Gefäßes deutlich gesenkt werden (siehe M.C. Morice et al., New England Journal of Medicine 2002 (346) 1773-1780).

Medikamentenbeschichtete Stents werden unter anderem in der Offenlegungsschrift US 2005/0019404 A1 offenbart.

In jüngeren Untersuchungen wurde allerdings festgestellt, dass bei Patienten, denen nach Stenose eines Herzkranzgefäßes ein medikamentenbeschichteter Stent implantiert wurde, die Mortalitätsrate höher ist als bei Patienten, die mit einem unbeschichteten Metallstent behandelt wurden. Quantitativ ergab diese Untersuchung, dass die Todeswahrscheinlichkeit durch Herzinfarkt in dem Zeitraum zwischen 6 Monaten und 3 Jahren nach der Stentimplantation bei der erstgenannten Patientengruppe um 32 % höher ist als bei der letztgenannten (siehe B. Lagerqvist et al., New England Journal of Medicine 2007 (356) 1009-1019).

T. Shirota et al. beschreiben einen metallischen intravaskulären Stent, der mit einer photoreaktiven, vernetzten Gelatine beschichtet ist (Biomaterials 2003 (24) 2295-2302).

Die Aufgabe der vorliegenden Erfindung ist es, einen Stent zur Verfügung zu stellen, bei dem das Risiko einer Restenose vermindert ist, ohne dabei auf anti-proliferative Wirkstoffe zurückgreifen zu müssen.

Diese Aufgabe wird erfindungsgemäß gelöst durch einen Gefäßstent der eingangs genannten Art, umfassend einen Träger aus einem formstabilen Material, sowie eine oder mehrere, zumindest bereichsweise auf dem Träger angeordnete Lagen eines unter physiologischen Bedingungen resorbierbaren Materials auf der Basis von vernetzter Gelatine, wobei die Adhäsion zwischen dem Träger und der Lage und/oder zwischen einzelnen Lagen dadurch aufhebbar ist, dass (i) der Vernetzungsgrad der Gelatine innerhalb einer oder mehrerer Lagen in Richtung des Trägers abnimmt, dass (ii) der mittlere Vernetzungsgrad der Gelatine in den einzelnen Lagen in Richtung der dem Träger benachbarten Lage zunimmt, und/oder dass (iii) eine oder mehrere Lagen mit Fettsäureresten modifizierte Gelatine enthalten.

Durch diese Aufhebung der Adhäsion wird eine Ablösung einer oder mehrerer Lagen von dem erfindungsgemäßen Stent unter physiologischen Bedingungen begünstigt. Die physiologischen Bedingungen, denen der erfindungsgemäße Gefäßstent bei seiner Verwendung im menschlichen oder tierischen Körper ausgesetzt ist, d.h. insbesondere die im Blut vorherrschenden Bedingungen, können dabei durch so genannte physiologische Standardbedingungen definiert und *in vitro* nachgestellt werden. Unter physiologischen Standardbedingungen wird im Rahmen dieser Erfindung die Inkubation in PBS-Puffer (pH 7,2) bei 37 °C verstanden.

Bevorzugt sind eine oder mehrere Lagen des resorbierbaren Materials einzeln ablösbar. Unter der Ablösung einer Lage im Sinne der vorliegenden Erfindung wird dabei eine zumindest bereichsweise flächige Ablösung des die Lage bildenden resorbierbaren Materials von der darunter liegenden Lage bzw. von dem Träger verstanden. Mit anderen Worten kommt es zu einer Ablösung von Bereichen oder Bruchstücken der Lage, die noch eine gewisse strukturelle Integrität aufweisen. Dieser Ablösevorgang ist im Gegensatz zu einer kontinuierlichen Degradation bzw. Resorption der Lage auf molekularer Ebene zu sehen, bei dem die Lage im Wesentlichen vollständig resorbiert wird, bevor eine Ablösung im oben beschriebenen Sinne erfolgt.

Die vorteilhafte Wirkung des erfindungsgemäßen Stents beruht darauf, dass durch die Aufhebung der Adhäsion und die Ablösung einer oder mehrerer Lagen des resorbierbaren Materials von dem Gefäßstent auch Zellen, Gewebe oder Thromben, die sich auf der Oberfläche des Stents gebildet haben, wieder von dem Stent abgelöst und mit dem Blutstrom aus dem betroffenen Gefäßbereich entfernt werden. Somit führt dieser Vorgang zu einer Reinigung des Stents und einer Erneuerung seiner Oberfläche, sodass eine Restenose verhindert oder zumindest verzögert wird, ohne dass ein Einsatz von Antiproliferativa oder ähnlichen Wirkstoffen erforderlich ist.

Die zunächst vorhandene Adhäsion zwischen der Lage und dem Träger bzw. den Lagen untereinander ist für die vorliegende Erfindung von Bedeutung, damit über die steuerbare Aufhebung der Adhäsion zeitlich gezielt und vorherbestimmbar die Ablösung von flächigen Lagenteilen ermöglicht ist und der Stent bis zum Beginn des Ablösens definierte Eigenschaften aufweist.

Die erfindungsgemäß geschaffene Möglichkeit der Aufhebung der Adhäsion zwischen einzelnen Lagen kann durch verschiedene Maßnahmen erreicht werden. Zum einen können zwischen einzelnen Lagen des resorbierbaren Materials Haftschichten aus einem unter physiologischen Bedingungen löslichen Material vorgesehen sein. Für solche Haftschichten sind verschiedene Materialien denkbar, beispielsweise niedermolekulares, lösliches Kollagenhydrolysat.

Bevorzugte Träger weisen eine mikroskopisch glatte, geschlossene Oberfläche auf. Damit wird verhindert, dass sich beim Aufbringen der Lagen auf dem Träger Material in Poren ablagert und damit eine Haftung aufgrund von Formschlusseffekten ergibt. Dies gilt insbesondere auch bei der Verwendung von Trägern in der Form von röhrchenartigen Gittergerüsten, die sich auch bei der vorliegenden Erfindung besonders eignen. Bei Trägern mit Gittergerüststruktur wird im Rahmen der besondere Vorteil erzielt, dass beim flächenmäßigen Ablösen einer Lage keine physiologisch zu großen und damit medizinisch bedenklichen Lagenstücke in das zirkulierende Blut abgegeben werden.

Bei Trägern mit Gitterstruktur wird bevorzugt eine Beschichtung von dessen Stegen angestrebt, die im Querschnitt von der bzw. den Lagen vorzugsweise vollständig umgeben sind. Auch nach der Beschichtung der Stege des Trägers bleiben die Zwischenräume zwischen den Stegen bevorzugt offen.

Eine Expansion des Stents ist dann möglich ohne die Integrität der Lage(n) auf dem Träger, d.h. der Lage(n) auf den Stegen des Trägers zu gefährden.

Des Weiteren können zwischen einzelnen Lagen des resorbierbaren Materials auch Trennschichten vorgesehen sein, worauf weiter unten noch im Detail eingegangen wird.

Die Aufhebung der Adhäsion zwischen einzelnen Lagen des resorbierbaren Materials (bzw. zwischen dem Träger und der benachbarten Lage) kann insbesondere auch auf einer zumindest partiellen Degradation des Materials unter physiologischen Bedingungen basieren, wobei unter einer Degradation in erster Linie diejenigen Vorgänge zu verstehen sind, die letztlich auch zu der Resorption des Materials führen. Die Degradationseigenschaften des resorbierbaren Materials können dabei durch verschiedene Maßnahmen gezielt beeinflusst werden, z.B. durch den Einsatz von Gelatinen mit unterschiedlichen Molekulargewichten und/oder die Beimischung weiterer Biopolymere zu dem Gelatine basierenden Material.

Gemäß einer bevorzugten Ausführungsform der Erfindung ist die Adhäsion in Abhängigkeit vom Vernetzungsgrad der Gelatine aufhebbar. Ein höherer Vernetzungsgrad der Gelatine führt zu einer langsameren Degradation (und Resorption) des Gelatine basierenden Materials, sodass durch diesen Parameter auf einfache Weise das Ablöseverhalten einzelner Lagen eingestellt werden kann.

Besonders bevorzugt ist die Gelatine in der Weise vernetzt, dass der Vernetzungsgrad innerhalb einer oder mehrerer Lagen des resorbierbaren Materials in Richtung des Trägers abnimmt. Durch einen geringeren Vernetzungsgrad der Gelatine an der dem Träger zugewandten Seite einer Lage wird bewirkt, dass es in diesem Bereich zu einer schnelleren Degradation des Materials kommt und dadurch die Adhäsion an den Träger (bzw. an die darunter liegende Lage) aufgehoben wird, während an der Außenseite der Lage durch den höheren Vernetzungsgrad der Gelatine eine gewisse strukturelle Integrität erhalten bleibt und die oben beschriebene, zumindest bereichsweise flächige Ablösung der Lage erreicht wird.

Bei dem formstabilen Material, aus dem der Träger gebildet ist, handelt es sich bevorzugt um ein unter physiologischen Bedingungen inertes Material, insbesondere um Metall und/oder Kunststoff. Als Träger können insbesondere expandierbare Gittergerüste oder ähnliche Strukturen in Röhrchen- oder Schlauchform zum Einsatz kommen.

Der erfindungsgemäße Gefäßstent kommt bevorzugt im kardiovaskulären Bereich, und hier insbesondere zur Behandlung der Herzkranzgefäße (koronarer Stent) zum Einsatz, da hier Gefäßstenosen zu einem Herzinfarkt führen können. Ebenso kann der Stent jedoch zur Behandlung von Stenosen in anderen Bereichen des Körpers verwendet werden.

Der erfindungsgemäße Effekt, die Gefahr von Restenosen zumindest zu reduzieren, kann bereits mit einer bereichsweisen Anordnung der Lage(n) auf dem Träger erreicht werden. Bevorzugt ist jedoch, wenn die mindestens eine Lage ca. 75 % oder mehr, insbesondere ca. 90 % oder mehr der Oberfläche des Trägers abdeckt. Besonders bevorzugt deckt die mindestens eine Lage im Wesentlichen die gesamte Oberfläche des Trägers ab.

Der erfindungsgemäße Einsatz von Gelatine als Basismaterial für die Lage(n) des resorbierbaren Materials bietet den Vorteil, dass es sich hierbei um ein sehr gut körperverträgliches, im Wesentlichen vollständig resorbierbares Produkt handelt, welches in reproduzierbarer Reinheit und Qualität hergestellt werden kann.

Darüber hinaus hat Gelatine im Rahmen der vorliegenden Erfindung insofern eine besonders vorteilhafte Wirkung, als sie die Angiogenese, d.h. die Neubildung von Blutgefäßen, fördert. Diesbezügliche Untersuchungen haben gezeigt, dass bei Einbringung von Gelatine enthaltenden Formkörpern in den menschlichen oder tierischen Körper ein lokaler Angiogenese fördernder Effekt auftritt. Dies gilt nicht nur für poröse Formkörper, bei denen ein Einwachsen von Kapillaren in die poröse Struktur beobachtet wird (siehe die deutsche Patentanmeldung 10 2005 054 937), sondern insbesondere auch für nicht-poröse Formkörper, wie z.B. Folien, bei denen die Angiogenese fördernde Wirkung in der Umgebung des Formkörpers beobachtet wird.

Es wurde festgestellt, dass die eingangs erwähnte höhere Mortalität bei medikamentenbeschichteten Stents ihre Ursache in erster Linie in einer unerwünschten Nebenwirkung der in den beschichteten Stents eingesetzten antiproliferativen Wirkstoffe hat, nämlich einer Verhinderung der Angiogenese im Umfeld des betroffenen Gefäßes. Im Fall einer Stenose besteht eine natürliche Reaktion des Körpers darin, durch die Neubildung von Blutgefäßen den Gefäßverschluss zu überbrücken. Dieser Vorgang wird durch Antiproliferativa o.ä. gehemmt, sodass im Fall einer dennoch eintretenden Restenose keine kollateralen Blutgefäße zur Verfügung stehen und ein Herzinfarkt die Folge ist (P. Meier et al., Journal of American Cardiology 2007 (49) 15 bis 20).

Durch die Möglichkeit des Verzichts auf anti-proliferative Wirkstoffe bei dem erfindungsgemäßen Stent wird nicht nur eine Angiogenese hemmende Wirkung vermieden, sondern es kommt im Gegenteil durch das Gelatine basierende Material zu einer Stimulierung der Angiogenese.

Der erfindungsgemäße Gefäßstent vermindert oder verzögert somit einerseits das Risiko einer Restenose durch den Selbstreinigungseffekt der Stentoberfläche, andererseits fördert er gleichzeitig die Bildung von kollateralen Blutgefäßen aufgrund der Angiogenese fördernden Wirkung der Gelatine in der Umgebung des betroffenen Gefäßes. Kommt es also dennoch zu einer Restenose, insbesondere nachdem sämtliche Lagen des Stents abgelöst sind, stehen kollaterale Blutgefäße als natürlicher Umgehungskreislauf zur Verfügung.

Ein Verzicht auf anti-proliferative Agenzien hat im Übrigen noch weitere Vorteile. So verhindern diese Wirkstoffe nicht nur die Anlagerung von Gewebe, welches zu einer Restenose führen kann, sondern auch eine Endothelialisierung des Stents. Bei der Endothelialisierung entsteht um den Stent eine Bindegewebsschicht, die mit den Komponenten des Blutes verträglich ist, wodurch die Aktivierung von Blutplättchen und Gerinnungsfaktoren am Stentmaterial verhindert wird, d.h. dieser Vorgang wirkt einer Thrombose im Bereich des Stents entgegen.

Bevorzugt ist das erfindungsgemäße resorbierbare Material zu einem überwiegenden Anteil aus vernetzter Gelatine gebildet. Dies bedeutet, dass die Gelatine den größten Anteil bei eventuell weiteren verwendeten Komponenten des Materials stellt.

Besonders geeignete Gelatinetypen sind Schweineschwartengelatine, die vorzugsweise hochmolekular ist und einen Bloomwert von ca. 160 bis ca. 320 g aufweist.

Um eine optimale Bioverträglichkeit des erfindungsgemäßen Stents zu gewährleisten, wird als Ausgangsmaterial bevorzugt eine Gelatine mit einem besonders geringen Gehalt an Endotoxinen eingesetzt. Bei Endotoxinen handelt sich um Stoffwechselprodukte oder Bruchstücke von Mikroorganismen, welche in dem tierischen Rohmaterial vorkommen. Der Endotoxingehalt von Gelatine wird in internationalen Einheiten pro Gramm (I.E./g) angegeben und gemäß dem LAL-Test bestimmt, dessen Durchführung in der vierten Ausgabe des Europäischen Arzneibuches (Ph. Eur. 4) beschrieben ist.

Um den Gehalt an Endotoxinen möglichst gering zu halten, ist es vorteilhaft, die Mikroorganismen möglichst frühzeitig im Zuge der Gelatineherstellung abzutöten. Ferner sollten entsprechende Hygienestandards beim Herstellungsprozess eingehalten werden.

Somit kann der Endotoxingehalt von Gelatine durch bestimmte Maßnahmen beim Herstellungsprozess drastisch gesenkt werden. Zu diesen Maßnahmen zählen in erster Linie die Verwendung frischer Rohmaterialien (z.B. Schweineschwarte) unter Vermeidung von Lagerzeiten, die sorgfältige Reinigung der gesamten Produktionsanlage unmittelbar vor Beginn der Gelatineherstellung sowie gegebenenfalls das Auswechseln von Ionenaustauschern und Filtersystemen in der Produktionsanlage.

Die im Rahmen der vorliegenden Erfindung eingesetzte Gelatine weist bevorzugt einen Endotoxingehalt von ca. 1.200 I.E./g oder weniger, noch mehr bevorzugt von ca. 200 I.E/g oder weniger auf. Optimalerweise liegt der Endotoxingehalt bei ca. 50 I.E./g oder weniger, jeweils gemäß dem LAL-Test bestimmt. Im Vergleich hierzu weisen manche handelsübliche Gelatinen Endotoxingehalte von über 20.000 I.E./g auf.

Durch die erfindungsgemäße Vernetzung der an sich löslichen Gelatine wird diese in ein unlösliches, jedoch unter physiologischen Bedingungen resorbierbares Material überführt. Dabei ist die Resorptions- bzw. Degradationsgeschwindigkeit des Materials vom Vernetzungsgrad der Gelatine abhängig und kann über einen relativ weiten Bereich eingestellt werden, wie dies bereits oben angesprochen wurde. Insbesondere kann der Zeitpunkt der Ablösung einzelner Lagen über den jeweiligen Vernetzungsgrad der Gelatine vorgegeben werden.

Formkörper auf Basis von vernetzter Gelatine, deren Resorptionseigenschaften und Herstellung sind in der Offenlegungsschrift DE 10 2004 024 635 A1 beschrieben.

Die Vernetzung der Gelatine kann sowohl durch chemische als auch durch enzymatische Vernetzungsmittel erfolgen. Unter den chemischen Vernetzungsmitteln ist eine Vernetzung unter Verwendung von Formaldehyd bevorzugt, welches gleichzeitig eine Sterilisierung bewirkt.

Ein bevorzugtes enzymatisches Vernetzungsmittel ist das Enzym Transglutaminase.

Bei der Herstellung von Formkörpern auf Basis von vernetzter Gelatine bietet sich ein zweistufiges Vernetzungsverfahren an, bei dem in einer ersten Stufe die Gelatine in Lösung partiell vernetzt wird. Aus der partiell vernetzten Gelatinelösung wird dann ein Formkörper hergestellt und einem zweiten Vernetzungsschritt unterworfen.

Der zweite Vernetzungsschritt kann insbesondere durch Einwirkung eines Vernetzungsmittels in der Gasphase, vorzugsweise Formaldehyd, durchgeführt werden.

Entsprechend diesem Verfahren kann der erfindungsgemäße Gefäßstent in der Weise hergestellt werden, dass eine partiell vernetzte Lösung des Gelatine basierenden Materials auf die Oberfläche des Trägers aufgebracht wird, z.B. durch Eintauchen des Trägers in die Lösung. Nach dem Trocknen der Lösung wird ein Träger mit einer Lage des resorbierbaren Materials erhalten, der dann dem zweiten Vernetzungsschritt, z.B. mit Formaldehyd in der Gasphase, unterworfen wird.

Durch den zweiten Vernetzungsschritt kann hierbei auf einfache Weise der bevorzugte Gradient im Vernetzungsgrad erhalten werden, da das Vernetzungsmittel im Wesentlichen nur von der Außenseite der Lage her in diese eindringen kann, was zu einer stärkeren Vernetzung an der Außenseite und zu einer geringeren Vernetzung an der inneren, dem Träger zugewandten Seite, führt.

Es ist im Rahmen der Erfindung ebenso denkbar, keine Vernetzung der Gelatine in der Lösung vorzusehen, sondern nur einen einzigen Vernetzungsschritt nach dem Aufbringen der Lage auf den Träger durchzuführen.

Bevorzugt enthält das resorbierbare Material ein oder mehrer Weichmacher. Hierdurch wird die Flexibilität des Materials erhöht, was insbesondere im Hinblick auf die Expansion des Stents nach dem Implantieren von Vorteil sein kann. Durch eine ausreichende Flexibilität kann weitgehend vermieden werden, dass die mindestens eine Lage beim Expandieren des Stents beschädigt wird oder sich mechanisch von dem Träger ablöst.

Bevorzugte Weichmacher sind beispielsweise Glycerin, Oligoglycerine, Oligoglycole, Sorbit und Mannit. Der Anteil an Weichmacher in dem resorbierbaren Material beträgt bevorzugt ca. 12 bis ca. 40 Gew.-%, weiter bevorzugt ca. 16 bis ca. 25 Gew.-%.

Gemäß einer bevorzugten Ausführungsform der Erfindung sind auf dem Träger mehrere Lagen des resorbierbaren Materials angeordnet. Durch das Vorsehen mehrerer Lagen, die sich bevorzugt jeweils einzeln ablösen, kann die Oberfläche des erfindungsgemäßen Stents wiederholt von Ablagerung befreit werden und so der Zeitraum, in dem die Gefahr der Restenose verringert wird, deutlich verlängert werden. Bevorzugt umfasst der Gefäßstent zwei bis fünf Lagen des resorbierbaren Materials.

Erfindungsgemäße Stents mit mehreren Lagen können auf relativ einfache Weise hergestellt werden, indem die oben dargestellten Verfahrensschritte (Aufbringen der ggf. partiell vernetzten Gelatinelösung, Trocknen und zweite Vernetzung) mehrmals nacheinander durchgeführt werden.

Bevorzugt nimmt der Vernetzungsgrad der Gelatine innerhalb jeder Lage in Richtung des Trägers ab. Dadurch wird die Aufhebung der Adhäsion bei der Ablösung jeder einzelnen Lage begünstigt.

Vorteilhafterweise sind die einzelnen Lagen des resorbierbaren Materials nacheinander von außen nach innen ablösbar. Eine solche sukzessive Ablösung wird bereits dadurch begünstigt, dass weiter innen befindliche Lagen durch die jeweils darüber liegenden Lagen in gewissem Ausmaß vor einer Degradation geschützt sind. Dies gilt auch dann, wenn sämtliche Lagen denselben mittleren Vernetzungsgrad der Gelatine aufweisen.

Bevorzugt ist jedoch, dass der mittlere Vernetzungsgrad der einzelnen Lagen in Richtung der dem Träger benachbarten Lage zunimmt. Durch einen hohen Vernetzungsgrad der inneren Lagen kann deren Degradation und Ablösung zusätzlich verzögert werden. Darüber hinaus kann das Ablöseverhalten der einzelnen Lagen gezielt an die jeweiligen Bedürfnisse angepasst werden, d.h. insbesondere an die erwartete Intensität der Ablagerung bzw. Gewebebildung an dem Stent. Im Idealfall ist die Zeit bis zur Ablösung der innersten Lage ausreichend lang, sodass bis zu diesem Zeitpunkt die Ausbildung von kollateralen Blutgefäßen, begünstigt durch den Angiogenese fördernden Effekt des Gelatine basierenden Materials, ermöglicht wird.

Die mittleren Vernetzungsgrade der einzelnen Lagen können beispielsweise so gewählt werden, dass sich die äußerste Lage nach ca. 1 bis 2 Wochen und die innerste, dem Träger benachbarte Lage nach ca. 3 bis 6 Monaten ablöst. Die genannten Zeiträume sind dabei auf den Zeitpunkt bezogen, an dem der erfindungsgemäße Stent physiologische Bedingungen ausgesetzt wird, d.h. insbesondere in ein Blutgefäß eingesetzt wird.

Unterschiedliche Vernetzungsgrade der einzelnen Lagen können bei dem oben beschriebenen Herstellungsverfahren durch unterschiedliche Vernetzungsmittelkonzentrationen in der Gelatinelösung und/oder durch unterschiedliche Konzentrationen oder Einwirkzeiten des Vernetzungsmittels im zweiten Vernetzungsschritt realisiert werden.

Die Dicke der einzelnen Lagen des resorbierbaren Materials liegt bevorzugt im Bereich von ca. 5 bis ca. 50 µm.

Gemäß einer weiteren Ausführungsform der Erfindung sind zwischen mehreren Lagen des resorbierbaren Materials und/oder auf der Außenseite der Lage(n) ein oder mehrere Trennschichten angeordnet. Durch solche Trennschichten können verschiedene vorteilhafte Wirkungen erzielt werden.

Zum einen kann durch die Trennschichten die Adhäsion zwischen mehreren Lagen reduziert werden. Bei fortschreitender Degradation des Gelatine basierenden Materials in der jeweils äußersten Lage wird somit die Aufhebung der Adhäsion und die Ablösung dieser Lage durch die Trennschicht beschleunigt, wobei die Trennschicht selbst auf der Außenseite der darunter liegenden Lage verbleibt.

Bevorzugt wird für die Trennschichten ein resorbierbares Material eingesetzt. Vorzugsweise weist dieses Material eine längere Resorptionszeit auf als das Gelatine basierende Material in der mindestens einen Lage, sodass die Trennschicht auf der Außenseite einer Lage zum Zeitpunkt der Ablösung dieser Lage noch vorhanden ist.

Die Trennschichten können beispielsweise aus einem Trennwachs gebildet sein.

Durch eine auf der jeweils äußersten Lage angeordnete Trennschicht kann zudem erreicht werden, dass die Ablagerung von Zellen oder Gewebe auf der Oberfläche des Stents verringert wird, indem die Trennschicht eine adhäsionshemmende Wirkung gegenüber diesen Ablagerungen aufweist. Dies wirkt der Gefahr einer Restenose zusätzlich entgegen. Wenn das Ausmaß der Ablagerungen verringert ist, kann auch ein späterer Ablösezeitpunkt der Lage(n) des resorbierbaren Materials gewählt werden.

Vorzugsweise enthält die mindestens eine Trennschicht modifizierte Gelatine. Es wurde gefunden, dass durch eine chemische Modifikation von Gelatine eine adhäsionshemmende Wirkung gegenüber Zellen erreicht werden kann.

Gemäß einer weiteren Ausführungsform der Erfindung ist vorgesehen, dass die modifizierte Gelatine in einer oder mehreren Lagen des Gelatine basierenden Materials enthalten ist. Hierdurch kann ein vergleichbarer Effekt wie bei Trennschichten aus modifizierter Gelatine erzielt werden.

Während Trennschichten zu einem hohen Anteil oder im Wesentlichen vollständig aus modifizierter Gelatine gebildet sein können, ist der mögliche Anteil an modifizierter Gelatine in den Lagen insofern limitiert, als nachteilige Wirkungen auf die Vernetzbarkeit der Gelatine und auf das Degradationsverhalten vermieden werden sollten.

Bevorzugt handelt es sich bei der modifizierten Gelatine um eine mit Fettsäureresten modifizierte Gelatine. Ein Beispiel hierfür stellt die Modifikation von Gelatine mit Dodecenyl-succinat dar.

Die Modifikation erfolgt hierbei insbesondere an den freien Aminogruppen der Lysinreste der Gelatine. Vorzugsweise sind ca. 10 bis ca. 80 % der Lysinreste der modifizierten Gelatine mit Fettsäureresten modifiziert.

Eine weitere Möglichkeit, um eine adhäsionshemmende Wirkung zu erzielen, stellt eine anionische Modifikation der Gelatine dar, beispielsweise die Succinylierung von Seitenketten.

Diese und weitere Vorteile der Erfindung werden anhand der folgenden Beispiele unter Bezugnahme auf die Zeichnung näher erläutert. Es zeigen im Einzelnen:
- Figur 1:: Darstellung eines erfindungsgemäßen Stents in komprimiertem Zustand vor der Implantation;
- Figur 2:: Darstellung eines erfindungsgemäßen Stents in expandiertem Zustand nach der Implantation;
- Figur 3:: Darstellung eines Ausschnitts aus der Struktur eines erfindungsgemäßen Stents;
- Figur 4:: schematische Querschnittsdarstellung der Lagen eines erfindungsgemäßen Stents;
- Figuren 5 und 6:: Diagramme betreffend die adhäsionshemmende Wirkung von modifizierter Gelatine;
- Figur 7:: fotografische Darstellung der Blutgefäßbildung im subkutanen Gewebe einer Maus;
- Figuren 8a bis c:: fotografische Darstellungen der vermehrten Blutgefäßbildung bei Anwesenheit verschiedener Angiogenese fördernder Substrate auf der Basis von vernetzter Gelatine; und
- Figur 9:: fotografische Darstellung des zeitabhängigen Ablösesverhalten zweier Lagen eines Gelatine basierenden Materials.

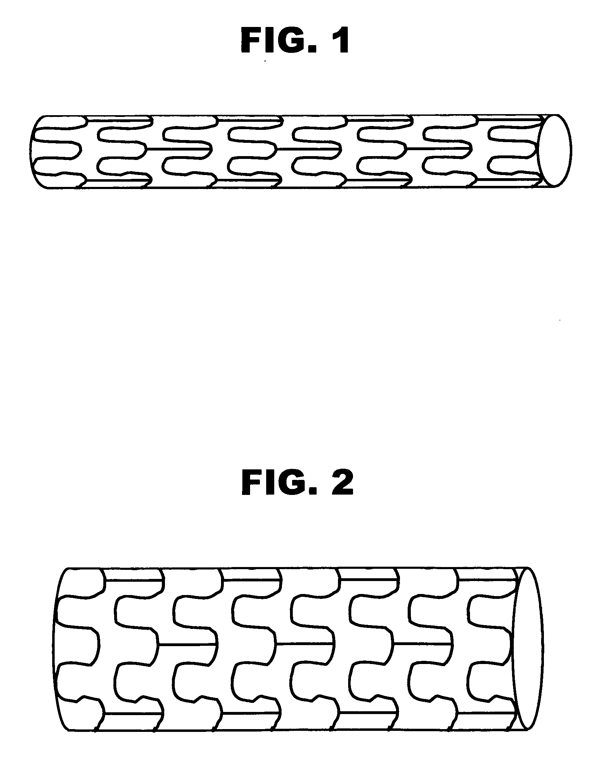

Die Figuren 1 und 2 zeigen ein Ausführungsbeispiel eines erfindungsgemäßen Gefäßstents, der insbesondere als koronarer Stent Verwendung findet. Der Stent umfasst einen Träger in Form eines röhrchenförmigen Gittergerüsts aus Metall oder Kunststoff, auf dessen Oberfläche mehrere Lagen eines resorbierbaren Material auf der Basis von vernetzter Gelatine angeordnet sind.

Figur 1 zeigt den Stent in einem komprimierten Zustand. Der Stent nimmt in diesem Zustand nur einen relativ geringen Querschnitt ein und kann daher in einen von einer Stenose betroffenen Gefäßbereich eingeführt werden.

Nach dem Implantieren wird der Stent z.B. mittels eines Ballon-Katheters geweitet, d.h. radial expandiert, sodass das betroffene Gefäß aufgeweitet und durch den Stent abgestützt wird. Dieser expandierte Zustand des Stents ist in der Figur 2 dargestellt. Sowohl das Gittergerüst des Trägers als auch die Lagen des resorbierbaren Materials weisen eine ausreichende Flexibilität auf, um diesen Expansionsvorgang durchführen zu können.

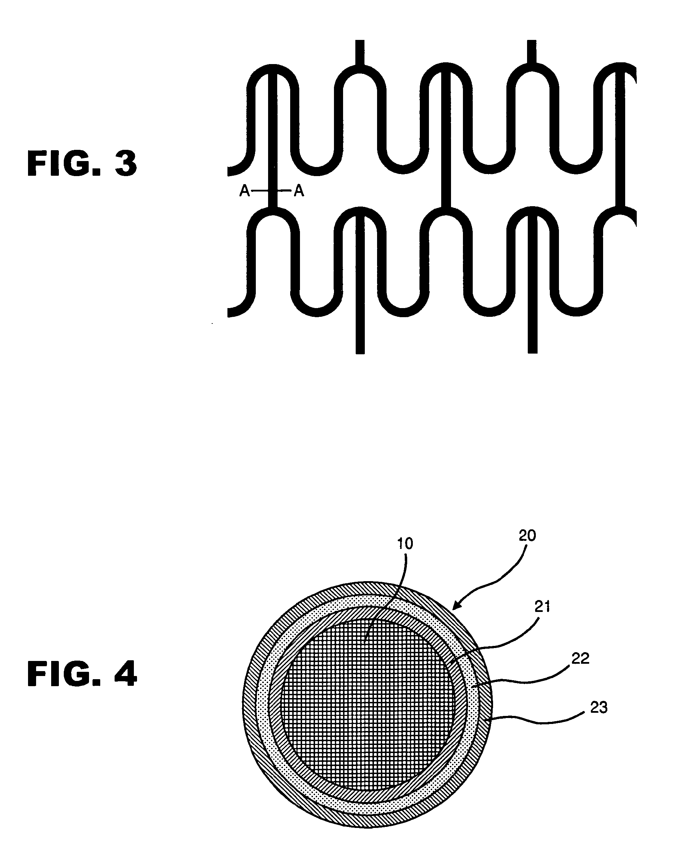

Die Figur 3 zeigt einen vergrößerten Ausschnitt aus der Gerüststruktur des erfindungsgemäßen Stents. Das Gittergerüst des Trägers wird aus einer Vielzahl von miteinander verbundenen Stegen gebildet, wobei die Lagen auf der Oberfläche dieser Stege angeordnet sind. Vorzugsweise ist hierbei ein möglichst großer Anteil der Gesamtoberfläche des Trägers abgedeckt.

Die in der Figur 3 gezeigte Geometrie der Gitterstruktur bzw. Anordnung der Stege ist hierbei lediglich beispielhaft. Die Gitterstruktur sollte jedoch so gestaltet sein, dass durch eine Verbiegung oder Verformung der Stege eine Expansion des Stents ermöglicht wird.

Eine schematische Darstellung des Querschnitts durch einen Steg 10 des Trägers, z.B. entlang der Linie A-A, ist in der Figur 4 gezeigt. Der Steg, der z.B. einen Durchmesser im Bereich von 100 bis 200 µm aufweist, ist von drei Lagen umgeben, und zwar einer inneren, dem Steg 10 benachbarte Lage 21, einer mittleren Lage 22 sowie einer äußeren Lage 23. Alternativ zu dieser Ausführungsform können auch eine, zwei oder mehr als drei Lagen vorgesehen sein.

Sämtliche Lagen sind aus einem unter physiologischen Bedingungen resorbierbaren Material auf der Basis von vernetzter Gelatine gebildet. Zur Erhöhung der Flexibilität der Lagen kann das Material zusätzlich einen Weichmacher, z.B. Glycerin, umfassen.

Der Vernetzungsgrad der Gelatine nimmt bevorzugt innerhalb jeder der drei Lagen 21, 22 und 23 in Richtung auf den Steg 10 des Trägers ab. Dies bedeutet, dass z.B. die äußere Lage 23 an ihrer inneren, d.h. der mittleren Lage 22 zugewandten Seite einen niedrigeren Vernetzungsgrad aufweist als an ihrer Außenseite 20.

Durch diesen graduellen Vernetzungsgrad kommt es unter physiologischen Bedingungen an der Innenseite der Lage 23 zu einer schnelleren Degradation des Gelatine basierenden Materials und dadurch, nach einer vorgegebenen Zeit, zu einer Aufhebung der Adhäsion an die Lage 22 und einer zumindest bereichsweise flächigen Ablösung der Lage 23 von der Lage 22. Dabei werden Ablagerungen von Zellen oder Gewebe, die sich an der Außenseite 20 der Lage 23, d.h. an der Oberfläche des Stents, gebildet haben, mit dem Blutstrom aus dem betroffenen Gefäßbereich entfernt. Nach dieser Ablösung bildet die Lage 22 die äußere Lage, sodass sich der eben beschriebene Vorgang bei dieser Lage wiederholen kann.

Der mittlere Vernetzungsgrad der Gelatine in den einzelnen Lagen 21, 22 und 23 nimmt bevorzugt in Richtung des Stegs 10 zu, d.h. die Lage 23 weist den geringsten und die Lage 21 den höchsten mittleren Vernetzungsgrad auf.

Beispielsweise sind die Vernetzungsgrade in den einzelnen Lagen so gewählt, dass sich die Lage 23 ca. ein bis zwei Wochen, die Lage 22 ca. vier bis acht Wochen und die Lage 21 ca. drei bis sechs Monaten nach dem Einführen des Gefäßstents in ein Blutgefäß ablöst.

Die einzelnen Lagen 21, 22 und 23 weisen vorzugsweise eine Dicke im Bereich von ca. 5 bis ca. 50 µm auf.

Zwischen den einzelnen Lagen 21, 22 und 23 und/oder auf der Außenseite 20 der Lage 23 können Trennschichten angeordnet sein. Solche Trennschichten können zum einen die Aufhebung der Adhäsion zwischen den einzelnen Lagen beschleunigen und/oder der Adhäsion von Zellen und Gewebe entgegenwirken.

Die Trennschichten und/oder die Lagen 21, 22 und 23 können insbesondere modifizierte Gelatine enthalten. Hierdurch kann die Zelladhäsion gegenüber nicht-modifizierter Gelatine herabgesetzt werden, wie in dem folgenden Beispiel 1 gezeigt wird.

### Beispiel 1: Hemmung der Zelladhäsion durch modifizierte Gelatine

Die Aminogruppen der Lysinreste in der Gelatine lassen sich mittels Bernsteinsäureanhydrid in eine succinierte Form überführen, wodurch der pKₛ-Wert des Gelatinematerials von 8 bis 9, wie er für die unmodifizierte Gelatine gefunden wird, auf ca. 4 abgesenkt wird.

Eine weitere Möglichkeit die Gelatine zu modifizieren besteht darin, die Aminogruppen der Lysinreste in Dodecenyl-succinyl-Gruppen zu überführen. Der pKₛ-Wert wird hierbei auf ca. 5 abgesenkt und gleichzeitig findet eine leichte Hydrophobierung der Gelatine durch den Fettsäurerest statt.

In beiden Fällen nimmt die Zelladhäsion gegenüber einer solchermaßen behandelten Gelatine deutlich ab, was in den nachfolgend beschriebenen Versuchen am Beispiel von porcinen Chondrozyten gezeigt wird.

Der Umsetzungsgrad der Lysingruppen der modifizierten Gelatine beträgt vorzugsweise 30 % oder mehr. Im Falle der Dodecenyl-succinierten Gelatine reichen oft Umsetzungsgrade von 40 bis 50 % sehr gut aus, während bei der succinierten Gelatine eher eine 80 %ige bis nahezu vollständige Umsetzung der Lysingruppen beste Resultate liefert.

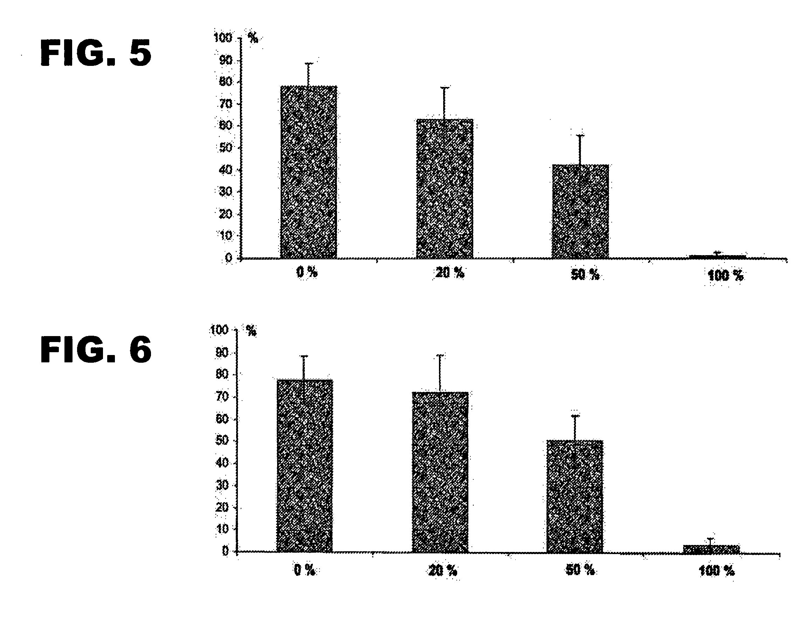

Die Figuren 5 und 6 zeigen Zelladhäsionsergebnisse für zu Testzwecken auf Glasoberflächen aufgebrachten Prüfflächen aus Gelatinematerialien, die ausgehend von Schweineschwartengelatine (MW 119 kDa) und einer zu ca. 95 % an den Lysingruppen succinierten Gelatine (Figur 5) bzw. ca. 45 % Dodecenyl-succinierten Gelatine (Figur 6) gleichen Typs hergestellt wurden. Es wurden jeweils Abmischungen von unmodifizierter Gelatine mit modifizierter Gelatine in den Verhältnissen 100:0, 80:20, 50:50 und 0:100 geprüft.

In den Tests wurden jeweils 20.000 porcine Chondrozyten auf einer Prüffläche während 4 h bei 37 °C inkubiert. Der Überstand wurde entfernt, die Oberfläche gewaschen und die auf der Oberfläche verbleibenden Zellen fixiert, um sie anschließend lichtmikroskopisch auszuwerten. Vergleichbare Ergebnisse wurden mit humanen Chondrozyten erhalten.

Die Prozentangaben in den Diagrammen stehen für den Anteil der auf den Folienprüfflächen gefunden Zellen gegenüber der zur Inkubation benutzten Anzahl, nachdem die vorstehend genannte Prozedur durchgeführt wurde.

Für beide modifizierte Gelatinearten ergaben sich Besiedelungseffekte nahe Null bei der ausschließlichen Verwendung von modifizierter Gelatine.

Diese deutliche Hemmung der Zelladhäsion durch chemisch modifizierte Gelatine kann im Rahmen der vorliegenden Erfindung ausgenutzt werden, um die Ablagerung von Zellen und Gewebe auf der jeweils äußeren Lage des Stents zu reduzieren.

Die bevorzugt zwischen den einzelnen Lagen angeordneten Trennschichten können dabei einen sehr hohen Anteil an modifizierter Gelatine von bis zu 100 % enthalten. Da diese Trennschichten nach der Ablösung der darüber befindlichen Lage jeweils die Oberfläche des Stents bilden, kann dadurch eine sehr starke adhäsionshemmende Wirkung des erfindungsgemäßen Stents erzielt werden kann.

### Beispiel 2 : Förderung der Angiogenese

Anhand des folgenden Beispiels soll die lokale Angiogenese fördernde Wirkung von Gelatine basierenden Materialien aufgezeigt werden.

### Herstellung von Folien aus einem Gelatine basierenden Material

Es wurden Gelatinefolien mit drei unterschiedlichen Vernetzungsgraden (Folien A, B und C) mittels eines zweistufigen Vernetzungsverfahrens hergestellt.

Für jeden der drei Ansätze wurden 25 g Schweineschwartengelatine (300 g Bloom), 9 g einer 85 Gew.-%igen Glycerinlösung sowie 66 g destilliertes Wasser gemischt und die Gelatine bei einer Temperatur von 60 °C gelöst. Nach dem Entgasen der Lösungen durch Ultraschall wurde zur Durchführung des ersten Vernetzungsschrittes eine wässrige Formaldehydlösung (2,0 Gew.-%ig, Raumtemperatur) zugegeben, und zwar 3,75 g dieser Lösung beim Ansatz A und jeweils 6,25 g der Lösung bei den Ansätzen B und C.

Die Mischungen wurden homogenisiert und bei ca. 60 °C in einer Dicke von ca. 250 µm auf eine Polyethylenunterlage gerakelt.

Nach dem Trocknen bei 30 °C und einer relativen Luftfeuchtigkeit von 30 % für etwa einen Tag wurden die Folien von der PE-Unterlage abgezogen und ca. 12 h unter denselben Bedingungen nachgetrocknet. Die getrockneten Folien (Dicke ca. 50 µm) wurden zur Durchführung des zweiten Vernetzungsschrittes in einem Exsikkator dem Gleichgewichtsdampfdruck einer 17 Gew.-%igen wässrigen Formaldehydlösung bei Raumtemperatur ausgesetzt. Im Falle der Folien A und B betrug die Einwirkzeit des Formaldehyddampfs 2 h, im Falle der Folie C 17 h.

Von den auf diese Weise hergestellten Formkörpern weist die Folie A insgesamt den geringsten und die Folie C insgesamt den höchsten Vernetzungsgrad auf, die Folie B liegt dazwischen. Dies spiegelt sich im unterschiedlichen Abbauverhalten der Folien wider, wobei die Resorptionszeiten der beschriebenen Folien unter physiologischen Bedingungen im Tierversuch (siehe unten) zwischen ca. 14 Tagen (Folie A) und ca. 21 Tagen (Folie C) liegen.

### Nachweis der Angiogenese fördernden Wirkung im Tierversuch

Die Angiogenese fördernde Wirkung der Gelatinefolien A, B und C *in vivo* wurde im Tierversuch untersucht. Als Versuchstiere wurden zehn Wochen alte Mäuse des Stammes Balb/C der Firma Charles River (Sulzfeld) mit einem Körpergewicht von 20 g verwendet.

Als Substrate wurden jeweils 5 x 5 mm² große Stücke der oben beschriebenen Gelatinefolien eingesetzt. Den Mäusen wurden jeweils zwei Folienstücke eines bestimmten Vernetzungsgrades subkutan im Nackenbereich implantiert. Hierzu wurden die Tiere narkotisiert und das Fell im Nackenbereich abrasiert. Mit einer Pinzette wurde ein Stück der Nackenhaut angehoben und ein Einschnitt von ca. 1 cm Länge durchgeführt. Über diesen Einschnitt wurde mit einer stumpfen Schere eine subkutane Tasche geschaffen, in die je zwei der Folienstücke mit einer Pinzette eingelegt wurden. Der Wundverschluss erfolgte mittels zweier Einzelknopfhefte.

Nach 12 Tagen wurden die Tiere getötet und die angiogenetische Wirkung der implantierten Substrate optisch ausgewertet.

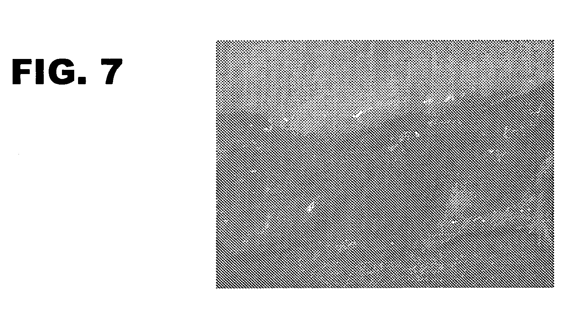

Die Figur 7 zeigt als Negativkontrolle den entsprechenden Bereich des subkutanen Gewebes einer Maus, bei der keine Implantation des Angiogenese fördernden Substrats durchgeführt wurde. Es ist eine nur sehr geringe Durchsetzung mit Blutgefäßen zu beobachten, wie es für das subkutane Hautgewebe der Maus normal ist.

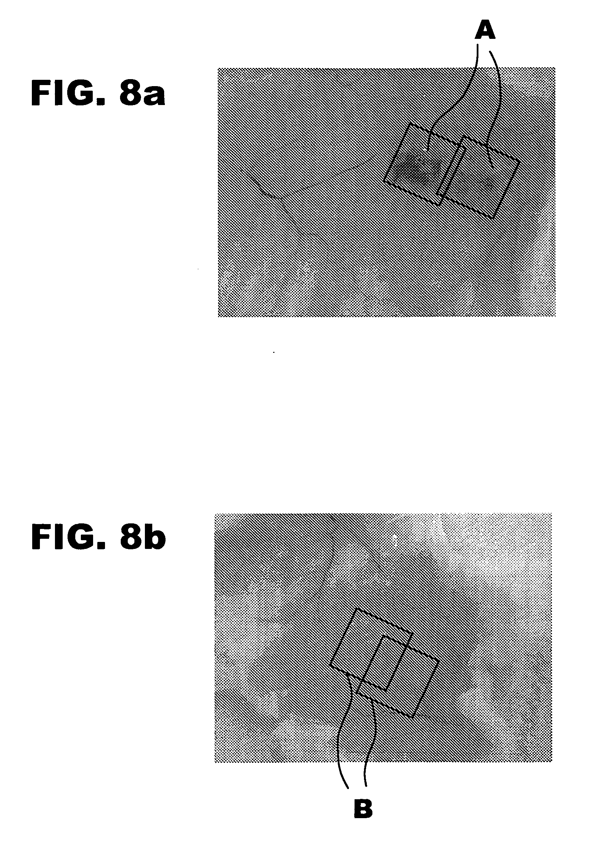

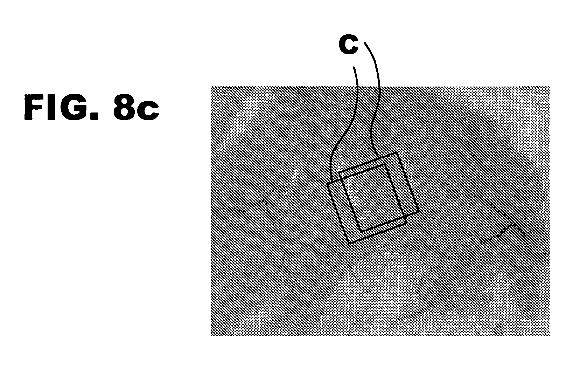

Die Figuren 8a bis 8c zeigen fotografische Aufnahmen des subkutanen Hautgewebes im Bereich der implantierten Folienstücke A, B bzw. C, nachdem die entsprechenden Mäuse 12 Tage nach der Implantation getötet wurden. Die Lage der Folienstücke ist durch schwarze Quadrate gekennzeichnet (Bezugszeichen A, B bzw. C für die entsprechende Folie), da die Folien selbst in der Fotografie schlecht sichtbar sind. Versuchsweise wurden die Folien z.T. mit Coomassie Brilliant Blue eingefärbt, wie dies in der Figur 8a zu erkennen ist.

In allen drei Abbildungen ist eine deutlich verstärkte Blutgefäßbildung in der Umgebung der implantierten Folienstücke zu erkennen. Sowohl die Anzahl als auch die Größe der Blutgefäße sind jeweils deutlich höher als bei der Negativkontrolle in Figur 7. Dieses Ergebnis belegt, dass durch ein unter physiologischen Bedingungen resorbierbares Material auf der Basis von vernetzter Gelatine die Angiogenese lokal stimuliert werden kann.

Dieser lokale Angiogenese fördernde Effekt von Materialien auf Basis von vernetzter Gelatine führt bei dem erfindungsgemäßen Gefäßstent zu einer besonders vorteilhaften Wirkung. Durch die Lagen des Gelatine enthaltende Materials wird die Bildung von kollateralen Blutgefäßen im Bereich des mit dem Stent behandelten Gefäßes stimuliert, wodurch im Fall einer Restenose, z.B. nach der vollständigen Ablösung sämtlicher Lagen, die Gefahr eines Herzinfarkts deutlich reduziert werden kann.

### Beispiel 3: Zeitabhängiges Ablöseverhalten mehrerer Lagen eines Gelatine basierenden Materials

Um das zeitabhängige Ablöseverhalten mehrerer Lagen eines Materials auf der Basis von vernetzter Gelatine qualitativ und quantitativ bestimmen zu können, wurde der im Folgenden beschriebene Versuch durchgeführt.

Zugunsten einer einfachen optischen Auswertungsmöglichkeit wurde hier als Träger kein Gittergerüst eines Stents, sondern eine ebene Unterlage aus Polyethylen verwendet, auf die zwei Lagen des resorbierbaren Materials großflächig aufgebracht wurden. Resorbierbare Materialien derselben Zusammensetzung können im Rahmen der vorliegenden Erfindung auf die Oberfläche eines Trägers eines erfindungsgemäßen Gefäßstents aufgebracht werden.

Um die zwei Lagen des resorbierbaren Materials in dem Versuch optisch unterscheiden zu können, wurde die erste Lage mit einem Weißpigment (Titandioxid) und die zweite Lage mit einem roten Lebensmittelfarbstoff (Candurin Wine Red) eingefärbt. Es wurden aus diesem Grund auch Lagen mit einer größeren Dicke hergestellt als die im Rahmen des erfindungsgemäßen Stents bevorzugten Dicken.

Ein erster Versuchsansatz 3-1 wurde wie folgt durchgeführt:
20 g Schweineschwartengelatine (300 g Bloom), 8 g Glycerin, 1 g Titandioxid und 69 g destilliertes Wasser wurden gemischt und die Gelatine 30 Minuten bei Raumtemperatur gequollen. Anschließend wurde die Gelatine durch Erwärmen der Mischung auf 60 °C gelöst, die Lösung homogenisiert und durch Ultraschall entgast.

Diese Gelatinelösung wurde in einer Dicke von ca. 550 µm auf einen ebenen Polyethylenträger gerakelt, um die erste Lage des resorbierbaren Materials auf dem Träger zu bilden.

Für die Durchführung einer Vernetzung der Gelatine wurde der Polyethylenträger mit der ersten Lage für 17 h im Exsikkator dem Gleichgewichtsdampfdruck einer 10 Gew.-%igen wässrigen Formaldehydlösung bei Raumtemperatur ausgesetzt.

Dadurch, dass der Formaldehyddampf im Wesentlichen nur von der dem Träger abgewandten Seite her in die Lage des resorbierbaren Materials eindringen kann, wird durch dieses Verfahren ein in Richtung des Trägers abnehmender Vernetzungsgrad der Gelatine erhalten.

Anschließend wurde die erste Lage des Gelatine basierenden Materials bei 26 °C und einer relativen Luftfeuchtigkeit von 10 % über Nacht getrocknet. Die getrocknete Lage wies eine Dicke von ca. 100 µm auf.

Der Träger mit der vernetzten ersten Lage wurde auf ca. 4 °C gekühlt. Zur Erzeugung einer Trennschicht wurde ein Boeson-Trennwachs auf die Lage aufgesprüht und mit einem Stofftuch gleichmäßig verteilt.

Die Gelatinelösung für die zweite Lage des resorbierbaren Materials wurde in derselben Weise hergestellt wie die Lösung für die erste Lage, wobei als Ausgangsmaterialien 20 g Gelatine, 4 g Glycerin, 73 g destilliertes Wasser und anstelle von Titandioxid 1 g Candurin Wine Red verwendet wurden.

Die resultierende Gelatinelösung wurde wiederum in einer Dicke von ca. 550 µm auf die mit dem Trennwachs versehene erste Lage des resorbierbaren Materials aufgetragen.

Die zweite Lage wurde ebenfalls einer Vernetzung mit Formaldehyddampf unterzogen, wie dies für die erste Lage beschrieben wurde, jedoch mit dem Unterschied, dass die Einwirkzeit des Vernetzungsmittels nur 2 h statt 17 h betrug. Dadurch weist die zweite Lage einen geringeren mittleren Vernetzungsgrad auf als die erste Lage, wobei der Vernetzungsgrad der Gelatine innerhalb der zweiten Lage ebenfalls in Richtung auf den Träger abnimmt.

Die Trocknung erfolgte wie für die erste Lage beschrieben. Nach dem Trocknen wies die zweite Lage eine Dicke von ca. 70 µm auf.

Das zeitabhängige Ablöseverhalten der zwei Lagen des Gelatine basierenden Materials unter physiologischen Bedingungen wurde durch Inkubation in PBS-Puffer (pH 7,2) bei 37 °C ermittelt. Durch diese physiologischen Standardbedingungen können die Bedingungen, wie sie beim Einsatz des erfindungsgemäßen Gefäßstents im Körper herrschen, nachgestellt werden.

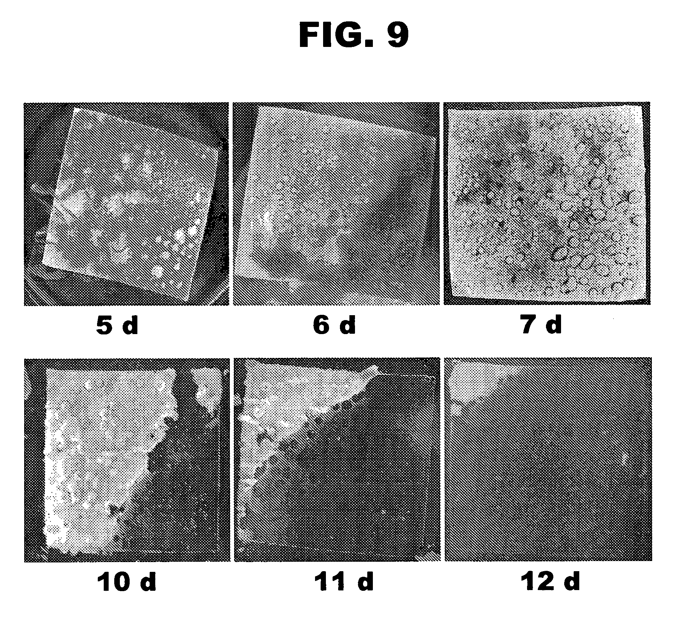

Die Figur 9 zeigt fotografische Darstellungen des Trägers mit den zwei Lagen gemäß dem Versuchsansatz 3-1 nach 5, 6, 7, 10, 11 und 12 Tagen Inkubation in dem PBS-Puffer.

Wie in den oberen drei Darstellungen zu erkennen ist, erfolgt zwischen dem 5. und 7. Tag der Inkubation die Aufhebung der Adhäsion der zweiten (äußeren) Lage und deren Ablösung von der darunter liegenden ersten Lage. Die im Original rot eingefärbte zweite Lage erscheint in den oberen drei Darstellungen der Figur 9 als dunkler Bereich, während die weiß eingefärbte erste Lage als deutlich hellerer Bereich zu erkennen ist. Nach 5 Tagen sind ca. 20 % der zweiten Lage abgelöst, es sind einzelne weiße Flecken der ersten Lage sichtbar. Nach 6 Tagen beträgt die Ablösung der zweiten Lage ca. 65 %, wobei die zweite Lage im Wesentlichen nur noch im rechten unteren Bereich des Trägers vorhanden ist. Nach 7 Tagen Inkubation ist die zweite Lage schließlich im Wesentlichen vollständig abgelöst. Auf der gesamten Fläche des Versuchsansatzes ist nun die erste Lage sichtbar, wobei diese zum Teil bereits aufgequollen ist und Blasen wirft, aber im Wesentlichen noch vollständig vorhanden ist.

Die Ablösung der ersten Lage des Gelatine basierenden Materials von dem Träger erfolgt im Wesentlichen zwischen dem 10. und 12. Tag der Inkubation, wie in den unteren drei Darstellungen der Figur 9 zu erkennen ist. Im Zuge der Ablösung der ersten Lage (weißer Bereich) wird der Polyethylenträger als dunkler Hintergrund sichtbar. Nach 10 Tagen sind ca. 35 %, nach 11 Tagen ca. 80 % und nach 12 Tagen Inkubation ca. 95 % der ersten Lage von dem Träger abgelöst.

Das beschriebene Ergebnis zeigt, dass es bei mehreren Lagen eines resorbierbaren Materials auf der Basis von vernetzter Gelatine möglich ist, durch unterschiedliche mittlere Vernetzungsgrade in den einzelnen Lagen eine Steuerung des Ablöseverhaltens dahingehend zu erreichen, dass die jeweils äußere Lage im Wesentlichen vollständig abgelöst ist, bevor die Ablösung der darunter liegenden Lage beginnt. Durch diesen Effekt kann bei dem erfindungsgemäßen Gefäßstent eine wiederholte Erneuerung der Stentoberfläche durch die sukzessive Ablösung mehrerer Lagen eines resorbierbaren Materials erzielt werden.

Des Weiteren zeigt der Versuch auch, dass durch die Aufhebung der Adhäsion zwischen den Lagen des Gelatine basierenden Materials eine zumindest bereichsweise flächige Ablösung einer Lage von der darunter liegenden Lage bzw. von dem Träger erfolgt. Dies wird durch einen geringeren Vernetzungsgrad der Gelatine an der Innenseite der jeweiligen Lage begünstigt.

Ein zweiter Versuchsansatz 3-2 wurde in derselben Weise durchgeführt wie der oben beschriebene Versuchsansatz 3-1, mit dem Unterschied, dass auf die Trennschicht (Boeson-Trennwachs) zwischen den beiden Lagen des Gelatine basierenden Materials verzichtet wurde. Bei diesem Ansatz wies die erste Lage eine Dicke von ca. 80 µm und die zweite Lage eine Dicke von ca. 100 µm auf.

Auch bei dem Versuchsansatz 3-2 wurde ein sequenzielles Ablöseverhalten der beiden Lagen beobachtet. Allerdings war in diesem Fall die erste Lage nach 6 Tagen Inkubation zu ca. 20 %, nach 7 Tagen Inkubation zu ca. 55 % und erst nach 10 Tagen Inkubation zu nahezu 100 % abgelöst.

Die zweite Lage des Ansatzes 3-2 war nach 13 Tagen noch im Wesentlichen vollständig erhalten. Nach 18 Tagen war sie zu ca. 20 % und nach 25 Tagen zu ca. 70 % abgelöst.

Dieses Ergebnis zeigt insbesondere, dass durch den Einsatz von Trennschichten die Ablösung einzelner Lagen von der darunter liegenden Lage, bei gleichem Vernetzungsgrad der Gelatine, beschleunigt werden kann (zweite Lage des Ansatzes 3-1 gegenüber dem Ansatz 3-2). Die spätere Ablösung der ersten Lage des Ansatzes 3-2 ist auf deren größere Dicke und Abschirmung durch die Trennschicht zurückzuführen.

Ein weiterer Versuchsansatz 3-3 wurde wie der Ansatz 3-2 durchgeführt, mit dem Unterschied, dass zu den Gelatinelösungen für die erste und zweite Lage vor dem Rakeln jeweils 2 ml einer 1 Gew.-%igen wässrigen Formaldehydlösung gegeben wurden. Durch diese zweistufige Vernetzung weisen beide Lagen dieses Ansatzes einen höheren mittleren Vernetzungsgrad auf im Vergleich zu den Ansätzen 3-1 und 3-2. Dies führte zu einem nochmals späteren Ablösezeitpunkt der ersten Lage dieses Ansatzes, die nach 25 Tagen Inkubation erst zu weniger als ca. 5 % von dem Träger abgelöst war. Eine vollständige Ablösung erfolgte erst nach 32 Tagen.

## Patentansprüche

1. Gefäßstent, insbesondere koronarer Stent, umfassend einen Träger aus einem formstabilen Material, sowie eine oder mehrere, zumindest bereichsweise auf dem Träger angeordnete Lagen eines unter physiologischen Bedingungen resorbierbaren Materials auf der Basis von vernetzter Gelatine, wobei die Adhäsion zwischen dem Träger und der Lage und/oder zwischen einzelnen Lagen dadurch aufhebbar ist, dass (i) der Vernetzungsgrad der Gelatine innerhalb einer oder mehrerer Lagen in Richtung des Trägers abnimmt, dass (ii) der mittlere Vernetzungsgrad der Gelatine in den einzelnen Lagen in Richtung der dem Träger benachbarten Lage zunimmt, und/oder dass (iii) eine oder mehrere Lagen mit Fettsäureresten modifizierte Gelatine enthalten.

2. Gefäßstent nach Anspruch 1, wobei eine oder mehrere Lagen des resorbierbaren Materials einzeln ablösbar sind.

3. Gefäßstent nach Anspruch 1 oder 2, wobei zwischen einzelnen Lagen des resorbierbaren Materials Haftschichten aus einem unter physiologischen Bedingungen löslichen Material angeordnet sind.

4. Gefäßstent nach einem der vorangehenden Ansprüche, wobei das resorbierbare Material zu einem überwiegenden Anteil aus vernetzter Gelatine gebildet ist.

5. Gefäßstent nach einem der vorangehenden Ansprüche, wobei das resorbierbare Material ein oder mehrere Weichmacher enthält.

6. Gefäßstent nach einem der vorangehenden Ansprüche, wobei der Gefäßstent mehrere Lagen, insbesondere zwei bis fünf Lagen, des resorbierbaren Materials umfasst.

7. Gefäßstent nach Anspruch 6, wobei der Vernetzungsgrad der Gelatine innerhalb jeder Lage in Richtung des Trägers abnimmt.

8. Gefäßstent nach einem der vorangehenden Ansprüche, wobei die Dicke der Lagen 5 bis ca. 50 µm beträgt.

9. Gefäßstent nach einem der vorangehenden Ansprüche, wobei zwischen mehreren Lagen des resorbierbaren Materials und/oder auf der Außenseite der Lage(n) ein oder mehrere Trennschichten angeordnet sind.

10. Gefäßstent nach Anspruch 9, wobei die mindestens eine Trennschicht eine längere Resorptionszeit aufweist als die Lage(n) des Gelatine basierenden Materials.

11. Gefäßstent nach Anspruch 9 oder 10, wobei die mindestens eine Trennschicht modifizierte Gelatine enthält.

## Claims

1. A vascular stent, in particular a coronary stent, comprising a carrier of a dimensionally stable material, as well as one or more layers, which are disposed at least in sections on the carrier, of a material based on crosslinked gelatin that is resorbable under physiological conditions, wherein the adhesion between the carrier and the layer and/or between individual layers can be neutralised, as a result of (i) the degree of crosslinking of the gelatin decreasing within one or more layers in the direction of the carrier, (ii) the average degree of crosslinking of the gelatin in the individual layers increasing in the direction of the layer adjacent to the carrier, and/or (iii) one or more layers containing gelatin modified with fatty acid groups.

2. The vascular stent according to claim 1, wherein one or more layers of the resorbable material are detachable individually.

3. The vascular stent according to claim 1 or 2, wherein adhesive layers of a material that is soluble under physiological conditions are disposed between individual layers of the resorbable material.

4. The vascular stent according to one of the preceding claims, wherein the resorbable material is formed predominantly of crosslinked gelatin.

5. The vascular stent according to one of the preceding claims, wherein the resorbable material contains one or more softening agents.

6. The vascular stent according to one of the preceding claims, wherein the vascular stent comprises a plurality of layers, in particular two to five layers, of the resorbable material.

7. The vascular stent according to claim 6, wherein the degree of crosslinking of the gelatin decreases within each layer in the direction of the carrier.

8. The vascular stent according to one of the preceding claims, wherein the thickness of the layers is ca. 5 to ca. 50 µm.

9. The vascular stent according to one of the preceding claims, wherein one or more separating layers are disposed between a plurality of layers of the resorbable material and/or on the outer side of the layer(s).

10. The vascular stent according to claim 9, wherein the at least one separating layer has a longer resorption time than the layer(s) of the gelatin-based material.

11. The vascular stent according to claim 9 or 10, wherein the at least one separating layer contains modified gelatin.

## Revendications

1. Stent vasculaire, en particulier stent coronarien, comprenant un support en un matériau rigide, ainsi qu'une ou plusieurs couches d'un matériau résorbable dans les conditions physiologiques à base de gélatine réticulée disposées au moins selon des domaines sur le support, où l'adhésion entre le support et la couche et/ou entre les différentes couches peut être neutralisée par le fait que (i) le degré de réticulation de la gélatine décroît à l'intérieur d'une ou plusieurs couches en direction du support, que (ii) le degré de réticulation moyen de la gélatine dans les différentes couches augmente en direction de la couche voisine du support, et/ou que (iii) une ou plusieurs couches contiennent de la gélatine modifiée avec des esters d'acides gras.

2. Stent vasculaire selon la revendication 1, où une ou plusieurs couches du matériau résorbable sont séparables individuellement.

3. Stent vasculaire selon la revendication 1 ou 2, où entre différentes couches du matériau résorbable sont disposées des couches adhésives en un matériau soluble dans les conditions physiologiques.

4. Stent vasculaire selon l'une des revendications précédentes, où le matériau résorbable est formé de gélatine réticulée en une fraction prépondérante.

5. Stent vasculaire selon l'une des revendications précédentes, où le matériau résorbable contient un ou plusieurs plastifiants.

6. Stent vasculaire selon l'une des revendications précédentes, où le stent vasculaire comprend plusieurs couches, en particulier deux à cinq couches, du matériau résorbable.

7. Stent vasculaire selon la revendication 6, où le degré de réticulation de la gélatine diminue à l'intérieur de chaque couche en direction du support.

8. Stent vasculaire selon l'une des revendications précédentes, où l'épaisseur des couches est 5 à environ 50 µm.

9. Stent vasculaire selon l'une des revendications précédentes, où entre plusieurs couches du matériau résorbable et/ou sur le côté externe de la couche ou des couches sont disposées une ou plusieurs couches de séparation.

10. Stent vasculaire selon la revendication 9, où la au moins une couche de séparation présente une plus grande durée de résorption que la ou les couches du matériau à base de gélatine.

11. Stent vasculaire selon la revendication 9 ou 10, où la au moins une couche de séparation contient de la gélatine modifiée.
